# EUROPEAN PATENT APPLICATION

(11) **EP 4 663 145 A1**
(43) Date of publication of application: **17.12.2025**
(21) Application number: 24223316.1
(22) Date of filing: 26.12.2024
(51) Int. Cl.: A61B 18/24

(54) **ABLATION CATHETER AND ABLATION SYSTEM**

(30) Priority: 12.06.2024 CN 202410753427
(71) Applicant: Hangzhou GenLight MedTech Co., Ltd., Hangzhou, Zhejiang (CN)
(72) Inventor: CAO, Peng, Hangzhou (CN); XIA, Liangdao, Hangzhou (CN)
(74) Representative: Monteiro Alves, Inês

(57) **Abstract**

The present disclosure provides an ablation catheter and an ablation system. The ablation catheter includes: an energy transmission element; an inner tube, a first channel being formed between an inner wall of the inner tube and an outer wall of the energy transmission element; an outer tube, a second channel being formed between an inner wall of the outer tube and an outer wall of the inner tube. The outer tube is provided with a hermetically sealed member at a proximal end, the second channel is in communication with the first channel in the hermetically sealed member, the first channel or the second channel is in communication with a cooling source, and a cooling medium from the cooling source passes through the first channel and the second channel and is discharged from the ablation catheter.

## Description

### TECHNICAL FIELD

The present disclosure relates to the field of medical equipment, in particular to an ablation catheter and an ablation system.

### BACKGROUND

Laser Interstitial Thermal Therapy (LITT) is a stereotactically-guided percutaneous minimally-invasive surgery in which a laser beam acts on a target via an energy transmission element to selectively ablate a tissue where a lesion occurs. In the surgery, optical energy is accurately transmitted by the energy transmission element to the tissue where the lesion occurs, so as to increase a temperature at a region where the lesion is located due to photothermal conversion, and achieve thermocoagulation and denaturation of the tissue, thereby to achieve a therapeutic effect.

In the above process, a corresponding ablation catheter needs to be used. In use, the energy transmission element is easily in contact with a sleeve structure, and the ablation catheter may be damaged due to excessive heat.

### SUMMARY

An object of the present disclosure is to provide an ablation catheter and an ablation system, so as to prevent an energy transmission element, an inner tube and an outer tube to be in contact with each other at a same position, thereby to prevent the ablation catheter from being damaged due to excessive heat.

In one aspect, the present disclosure provides in some embodiments an ablation catheter, including: an energy transmission element; an inner tube sleeved onto the energy transmission element, a first channel being formed between an inner wall of the inner tube and an outer wall of the energy transmission element; an outer tube sleeved onto the inner tube, a second channel being formed between an inner wall of the outer tube and an outer wall of the inner tube. The outer tube is provided with a hermetically sealed member at a proximal end, the second channel is in communication with the first channel in the hermetically sealed member, the first channel or the second channel is in communication with a cooling source, and a cooling medium from the cooling source passes through the first channel and the second channel and is discharged from the ablation catheter. In any cross section including the energy transmission element, the inner tube and the outer tube, any virtual line extending from a center of the energy transmission element to the outer wall of the outer tube passes through at least one of the first channel or the second channel.

In a possible embodiment of the present disclosure, in an initial state, the energy transmission element is arranged coaxially with the inner tube, no contact point is formed between the inner wall of the inner tube and the outer wall of the energy transmission element, a cross section of the energy transmission element has a shape different from a cross section of the inner wall of the inner tube, a plurality of first limiting gaps is arranged circumferentially between the inner wall of the inner tube and the outer wall of the energy transmission element, and the first limiting gap is located at a position where the inner wall of the inner tube is spaced apart from the outer wall of the energy transmission element by a smallest distance in the initial state.

In a possible embodiment of the present disclosure, in an initial state, the outer tube is arranged coaxially with the inner tube, no contact point is formed between the inner wall of the outer tube and the outer wall of the inner tube, a cross section of the outer wall of the inner tube has a shape different from a cross section of the inner wall of the outer tube, a plurality of second limiting gaps is arranged circumferentially between the inner wall of the outer tube and the outer wall of the inner tube, and the second limiting gap is located at a position where the inner wall of the outer tube is spaced apart from the outer wall of the inner tube by a smallest distance in the initial state.

In a possible embodiment of the present disclosure, the ablation catheter further includes: a first adapter assembly, the energy transmission element passing through the first adapter assembly; and a second adapter assembly, the inner tube passing through the second adapter assembly, a part of the outer tube extending into the second adapter assembly, the proximal end of the outer tube extending beyond a proximal end of the second adapter assembly, a distal end of the inner tube extending to a distal end of the second adapter assembly, and the distal end of the second adapter assembly being detachably coupled to a proximal end of the first adapter assembly. One of the first adapter assembly and the second adapter assembly is in communication with the cooling source, the cooling medium from the cooling source passes through the first channel and the second channel and is discharged from the ablation catheter via the other of the first adapter assembly and the second adapter assembly. A rotation limiting structure is provided between the second adapter assembly and the first adapter assembly, and configured to limit rotation of the second adapter assembly relative to the first adapter assembly.

In a possible embodiment of the present disclosure, the distal end of the second adapter assembly is inserted into the proximal end of the first adapter assembly, the proximal end of the first adapter assembly is provided with a guiding groove extending in a direction parallel to an axial direction of the first adapter assembly, an outer peripheral surface of the second adapter assembly is provided with a guiding protrusion adapted to the guiding groove, and the guiding protrusion is inserted into the guiding groove to limit the rotation of the second adapter assembly relative to the first adapter assembly.

In a possible embodiment of the present disclosure, the distal end of the second adapter assembly is inserted into the proximal end of the first adapter assembly, a first central guiding structure is arranged in a cavity of the first adapter assembly, a second central guiding structure is arranged at the distal end of the second adapter assembly, and in a state where the second adapter assembly is coupled to the first adapter assembly through a connection member, the second central guiding structure cooperates with the first central guiding structure in such a manner that a central axis of the second adapter assembly coincides with a central axis of the first adapter assembly.

In a possible embodiment of the present disclosure, the first adapter assembly includes: a first cylinder, the energy transmission element passing through the first cylinder, a first interface member extending from the first cylinder; a sealing plug; and a sealing cover configured to press the sealing plug against a distal end of the first cylinder. The energy transmission element passes through the sealing cover and the sealing plug, and is in hermetical engagement with the sealing plug.

In a possible embodiment of the present disclosure, the second adapter assembly includes: a second cylinder, the inner tube passing through the second cylinder, a part of the outer cylinder extending into the second cylinder, a second interface member extending from the second cylinder and in communication with the second channel; an inner tube sealing member arranged at a distal end of the second cylinder; an outer tube sealing member arranged at a proximal end of the second cylinder; and an outer tube fixation member configured to press the outer tube sealing member against the proximal end of the second cylinder, and couple the outer tube to the second cylinder coaxially.

In a possible embodiment of the present disclosure, the energy transmission element is coupled to a laser source, and a size of the ablation catheter is determined through obtaining diameter information about the outer tube and the inner tube based on laser energy of the laser source and target temperature information about the ablation catheter after determining a diameter of the energy transmission element.

In a possible embodiment of the present disclosure, the obtaining the diameter information about the outer tube and the inner tube based on the laser energy of the laser source and the target temperature information about the ablation catheter includes: obtaining a flow quantity corresponding to the target temperature information about the ablation catheter based on laser power of the laser source, the target temperature information and a first fitting model, the first fitting model including a mapping relation between temperature information about the ablation catheter and the laser power as well as the flow quantity; obtaining the diameter information about the outer tube based on the flow quantity and a second fitting model, the second fitting model including a mapping relation among the flow quantity, an intensity of pressure and the diameter information about the outer tube, the diameter information about the outer tube including an outer diameter and an inner diameter of the outer tube; and obtaining the dimeter information about the inner tube based on the outer diameter of the outer tube.

In a possible embodiment of the present disclosure, the energy transmission element includes a light-exiting member facing the hermetically sealed member, the temperature information about the ablation catheter includes an outer wall temperature of the outer tube and a core temperature of the light-exiting member, and the first fitting model includes a first sub-model and a second sub-model. The first sub-model is Tx1=31.92+4.424*Px-0.7383*Qx, where Tx1 represents the outer wall temperature of the outer tube, Px represents the laser power and Qx represents the flow quantity; and the second sub-model is Tx2=26.00+4.272*Px-0.3809*Qx, where Tx2 represents the core temperature of the light-exiting member, Px represents the laser power, and Qx represents the flow quantity.

In a possible embodiment of the present disclosure, the target temperature information includes a target temperature of the outer wall of the outer tube which is smaller than or equal to 90°C.

In a possible embodiment of the present disclosure, an effective flow area of the second channel is 1.1 to 1.2 times of an effective flow area of the first channel.

In another aspect, the present disclosure provides in some embodiments an ablation system, including: the above-mentioned ablation catheter; a cooling resource in communication with the first channel or the second channel of the ablation catheter; and a medium recycling pool in communication with the second channel or the first channel of the ablation catheter.

In a possible embodiment of the present disclosure, the ablation system further includes: a laser source configured to output a laser beam to the laser transmission element, so as to perform laser ablation on a to-be-ablated tissue; a temperature sensor configured to monitor in real time a temperature of the ablation catheter, so as to obtain target temperature information; a control unit configured to control the output of the laser beam and the supply of a cooling medium; and an adjustment unit configured to dynamically adjust in real time the supply of the cooling medium using a first fitting model in response to the target temperature information about the ablation catheter and the laser beam from the laser source, so as to control the temperature of the ablation catheter. The first fitting model includes a mapping relation between temperature information about the ablation catheter and laser power of the laser source as well as a flow quantity.

In a possible embodiment of the present disclosure, the energy transmission element includes a light-exiting member, the temperature information about the ablation catheter includes an outer wall temperature of the outer tube and a core temperature of the light-exiting member, and the first fitting model includes a first sub-model and a second sub-model. The first sub-model is Tx1=31.92+4.424*Px-0.7383*Qx, where Tx1 represents the outer wall temperature of the outer tube, Px represents the laser power and Qx represents the flow quantity; and the second sub-model is Tx2=26.00+4.272*Px-0.3809*Qx, where Tx2 represents the core temperature of the light-exiting member, Px represents the laser power, and Qx represents the flow quantity.

According to the ablation catheter and the ablation system in the embodiments of the present disclosure, the ablation catheter includes the energy transmission element, the inner tube and the outer tube, the first channel is formed between the inner wall of the inner tube and the outer wall of the energy transmission element, and the second channel is formed between the inner wall of the outer tube and the outer wall of the inner tube. In any cross section including the energy transmission element, the inner tube and the outer tube, any virtual line extending from the center of the energy transmission element to the outer wall of the outer tube passes through at least one of the first channel or the second channel. In a case that the ablation catheter is in an operating state, the cooling medium from the cooling source passes through the first channel and the second channel, and there is the cooling medium on any virtual line extending from the center of the energy transmission element to the outer wall of the outer tube, i.e., the cooling medium exists in any radial direction of the ablation catheter. In this way, it is able to reduce the temperature, and prevent the ablation catheter from being overheated in a case that the energy transmission element, the outer tube and the inner tube are in contact with each other at a same position, thereby to prevent the ablation catheter from being damaged, and improve a service life and reliability of the ablation catheter.

### BRIEF DESCRIPTION OF THE DRAWINGS

In order to illustrate the technical solutions of the present disclosure or the related art in a clearer manner, the drawings desired for the present disclosure or the related art will be described hereinafter briefly. Obviously, the following drawings merely relate to some embodiments of the present disclosure, and based on these drawings, a person skilled in the art may obtain the other drawings without any creative effort.
FIG. 1 is a sectional view of an ablation catheter according to one embodiment of the present disclosure;
FIG. 2 is a schematic view showing the ablation catheter applied to an ablation system according to one embodiment of the present disclosure;
FIG. 3 is a schematic view showing a cooling principle of the ablation catheter in an operating state according to one embodiment of the present disclosure;
FIGs. 4, 5 and 6 are sectional views of an energy transmission element, an inner tube and an outer tube of the ablation catheter according to one embodiment of the present disclosure;
FIGs. 7, 8 and 9 are sectional views of the energy transmission element, the inner tube and the outer tube of the ablation catheter according to one embodiment of the present disclosure;
FIG. 10 is an exploded view of a second adapter assembly and a first adapter assembly of the ablation catheter according to one embodiment of the present disclosure;
FIG. 11 is a diagram of predicted outer wall temperatures of the outer tube in a first sub-model obtained through multiple linear regression fitting using simulation software according to one embodiment of the present disclosure;
FIG. 12 is a diagram of residuals of outer wall temperatures of the outer tube in the first sub-model obtained through multiple linear regression fitting using simulation software according to one embodiment of the present disclosure;
FIG. 13 is a diagram of predicted core temperatures of a light-exiting member in a second sub-model obtained through multiple linear regression fitting using simulation software according to one embodiment of the present disclosure;
FIG. 14 is a diagram of residuals of the core temperatures of the light-exiting member in the second sub-model obtained through multiple linear regression fitting using simulation software according to one embodiment of the present disclosure; and
FIG. 15 is a fitted curve diagram of a second fitting model according to one embodiment of the present disclosure.

### Reference Sign List

- 100: ablation catheter
- 110: energy transmission element
- 120: inner tube
- 130: outer tube
- 131: hermetically sealed member
- 140: first adapter assembly
- C1: guiding groove
- D1: first central guiding structure
- 141: first cylinder
- 1411: first interface member
- 142: sealing plug
- 143: sealing cover
- 150: second adapter assembly
- C2: guiding protrusion
- D2: second central guiding structure
- 151: second cylinder
- 1511: second interface member
- 152: inner tube sealing member
- 153: outer tube sealing member
- 154: outer tube fixation member
- 160: connection member
- 200: cooling source
- 210: cooling source tank
- 220: pump
- 230: heater
- 300: medium recycling pool
- 400: inlet pipeline
- 500: outlet pipeline
- T1: first channel
- T2: second channel
- M1: first contact portion
- M2: second contact portion
- H1: first limiting gap
- H2: second limiting gap

### DETAILED DESCRIPTION

The present disclosure will be described hereinafter in a clear and complete manner in conjunction with the drawings and embodiments. Obviously, the following embodiments merely relate to a part of, rather than all of, the embodiments of the present disclosure, and based on these embodiments, a person skilled in the art may, without any creative effort, obtain the other embodiments, which also fall within the scope of the present disclosure.

It should be appreciated that, such words as "on/above", "under/below", "left", "right", "front" and "rear" in the embodiments of the present disclosure are merely use to describe relative positions or movements of members in a specific pose aa shown in the drawings, and in a case that the specific pose changes, the relative positions or the movements may change too.

In addition, such words as "first" and "second" are merely used to differentiate different components rather than to represent any order, number or importance, i.e., they are used to implicitly or explicitly indicate that there is at least one component. In addition, the technical solutions in the embodiments of the present disclosure may be combined in the case of no conflict.

Along with the development of the cutting-edge technology, the diagnosis and treatment of intracranial tumors have made a great progress, and a survival expectancy and a survival rate of patients with intracranial tumors have been improved significantly. Magnetic Resonance Imaging (MRI)-guided LITT is a stereotactically-guided percutaneous minimally-invasive surgery in which a laser beam acts on a target via an energy transmission element to selectively ablate a tissue where a lesion occurs. In the surgery, optical energy is accurately transmitted by the energy transmission element to the tissue where the lesion occurs, so as to increase a temperature at a region where the lesion is located due to photothermal conversion, and achieve thermocoagulation and denaturation of the tissue, thereby to achieve a therapeutic effect. Studies show that, in a case that a tissue temperature is greater than 60°C, rapid coagulative necrosis and instantaneous cell death occur; in a case that the tissue temperature is greater than 100°C, tissue gasification occurs; and in a case that the tissue temperature is greater than 300°C, tissue carbonization occurs. Due to the carbonized tissue, the transmission of the optical energy and the heat may be adversely affected, and the energy transmission element may be damaged. In addition, a gas generated in the tissue gasification at a high temperature becomes an insulator, and the heat accumulation may be adversely affected. Hence, it is necessary to prevent a too high ablation temperature.

The present disclosure provides in some embodiments an ablation catheter and an ablation system including the ablation catheter.

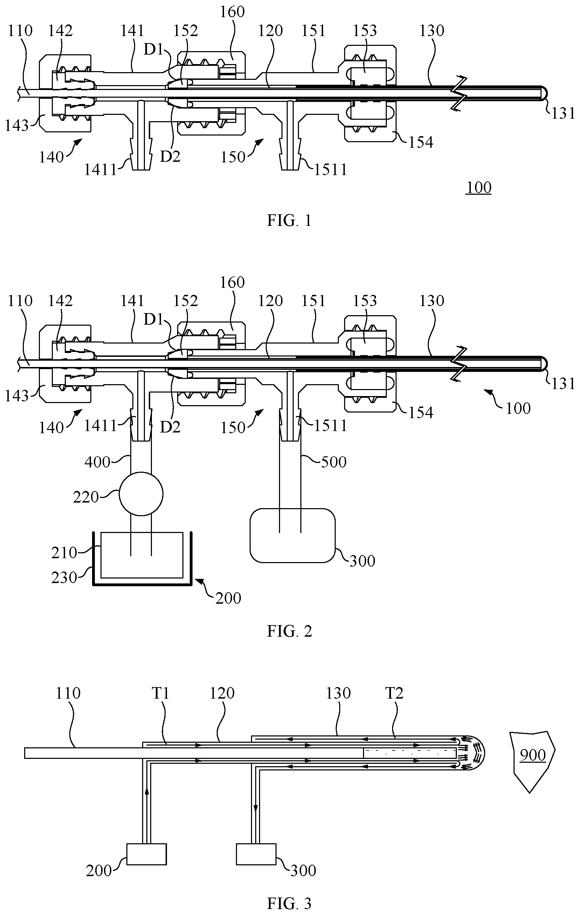

FIG. 1 is a sectional view of the ablation catheter according to one embodiment of the present disclosure, FIG. 2 is a schematic view showing the ablation catheter applied to the ablation system according to one embodiment of the present disclosure, and FIG. 3 is a schematic view showing a cooling principle of the ablation catheter in an operating state according to one embodiment of the present disclosure. The ablation catheter 100 includes an energy transmission element 110, an inner tube 120 and an outer tube 130.

For example, the energy transmission element 110 is an optical fiber for transmitting a laser signal. The inner tube 120 is sleeved onto the energy transmission element 110, and a first channel T1 is formed between an inner wall of the inner tube 120 and an outer wall of the energy transmission element 110. The outer tube 130 is sleeved onto the inner tube 120, a second channel T2 is formed between an inner wall of the outer tube 130 and an outer wall of the inner tube 120, the outer tube 130 is provided with a hermetically sealed member 131 at a proximal end, and the second channel T2 is in communication with the first channel T1 in the hermetically sealed member 131. The first channel T1 or the second channel T2 is in communication with a cooling source 200 for providing a cooling medium, e.g., a cooling liquid, and the cooling medium from the cooling source 200 passes through the first channel T1 and the second channel T2 and is then discharged from the ablation catheter. For example, the first channel T1 is in communication with the cooling source 200, and the cooling medium from the cooling source 200 sequentially passes the first channel T1 and the second channel T2 and is then discharged from the ablation catheter.

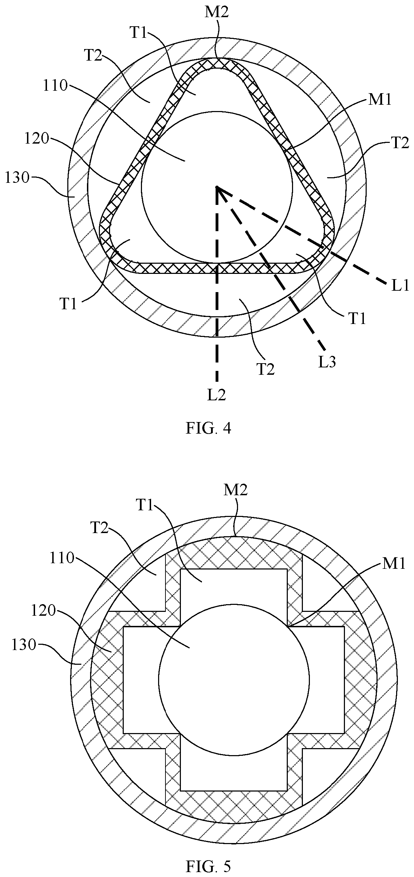

FIGs. 4, 5 and 6 are sectional views of the energy transmission element, the inner tube and the outer tube of the ablation catheter according to one embodiment of the present disclosure. In some embodiments of the present disclosure, in any cross section including the energy transmission element 110, the inner tube 120 and the outer tube 130, any virtual line extending from a center of the energy transmission element 110 to the outer wall of the outer tube 130 passes through at least one of the first channel T1 or the second channel T2.

FIG. 4 illustratively shows three typical virtual lines, i.e., a first virtual line L1, a second virtual line L2, and a third virtual line L3. The first virtual line L1 at least passes through the first channel T1, the second virtual line L2 at least passes through the second channel T2, and the third virtual line L3 passes through the first channel T1 and the second channel T2.

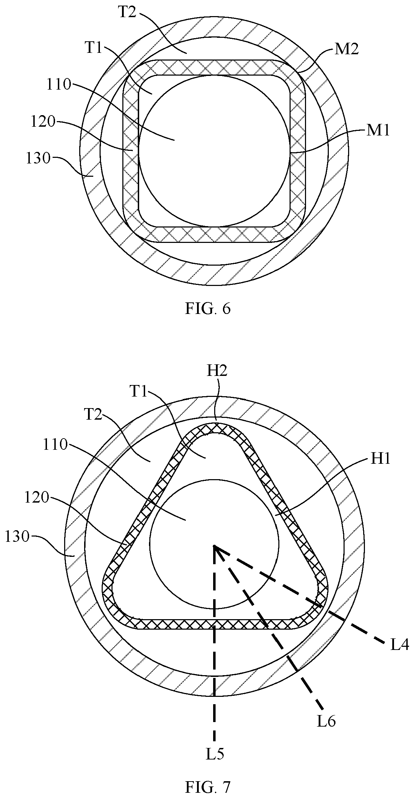

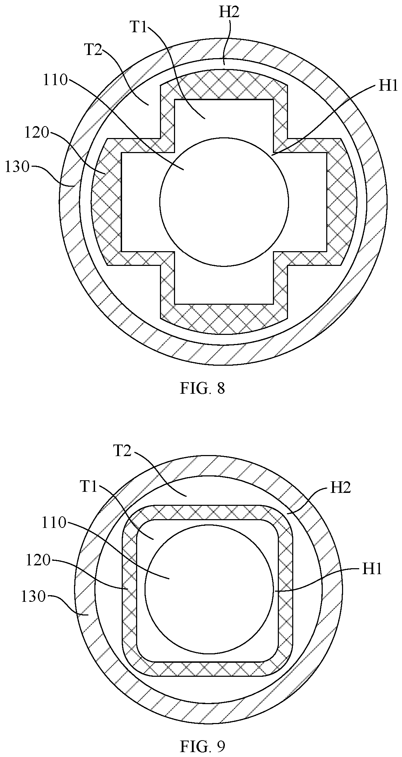

FIGs. 7, 8 and 9 are sectional views of the energy transmission element, the inner tube and the outer tube of the ablation catheter according to one embodiment of the present disclosure. FIG. 7 illustratively shows three typical virtual lines, i.e., a fourth virtual line L4, a fifth virtual line L5 and a sixth virtual line L6, which all pass through the first channel T1 and the second channel T2.

According to the ablation catheter 100 in the embodiments of the present disclosure, the ablation catheter 100 includes the energy transmission element 110, the inner tube 120 and the outer tube 130, the first channel T1 is formed between the inner wall of the inner tube 120 and the outer wall of the energy transmission element 110, and the second channel T2 is formed between the inner wall of the outer tube 130 and the outer wall of the inner tube 120. In any cross section including the energy transmission element 110, the inner tube 120 and the outer tube 130, any virtual line extending from the center of the energy transmission element 110 to the outer wall of the outer tube 130 passes through at least one of the first channel T1 or the second channel T2. In a case that the ablation catheter 100 is in an operating state, the cooling medium from the cooling source 200 passes through the first channel T1 and the second channel T2, and there is the cooling medium on any virtual line extending from the center of the energy transmission element 110 to the outer wall of the outer tube 130, i.e., the cooling medium exists in any radial direction of the ablation catheter 100. In this way, it is able to reduce the temperature, and prevent the ablation catheter 100 from being overheated in a case that the energy transmission element 110, the outer tube 130 and the inner tube 120 are in contact with each other at a same position, thereby to prevent the ablation catheter 100 from being damaged, and improve a service life and reliability of the ablation catheter 100.

As shown in FIGs. 4 to 6, in some embodiments of the present disclosure, a cross section of the energy transmission element 110 has a shape different from a cross section of the inner wall of the inner tube 120, and a plurality of first contact portions M1 is arranged circumferentially between the inner wall of the inner tube 120 and the outer wall of the energy transmission element 110.

The cross section of the energy transmission element 110 may be of a circular, oval or polygonal shape, e.g., a regular polygonal shape or an irregular polygonal shape. The cross section of the inner wall of the inner tube 120 may be of a circular, oval or polygonal shape.

In some embodiments of the present disclosure, a cross section of the outer wall of the inner tube 120 has a shape different from a cross section of the inner wall of the outer tube 130, and a plurality of second contact portions M2 is arranged circumferentially between the inner wall of the outer tube 130 and the outer wall of the inner tube 120.

The cross section of the outer wall of the inner tube 120 may be of a circular, oval or polygonal shape, and the cross section of the inner wall of the outer tube 130 may be of a circular, oval or polygonal shape.

As shown in FIGs. 7 to 9, in some embodiments of the present disclosure, in an initial state, the energy transmission element 110 is arranged coaxially with the inner tube 120, no contact point is formed between the inner wall of the inner tube 120 and the outer wall of the energy transmission element 110, a cross section of the energy transmission element 110 has a shape different from a cross section of the inner wall of the inner tube 120, a plurality of first limiting gaps H1 is arranged circumferentially between the inner wall of the inner tube 120 and the outer wall of the energy transmission element 110, and the first limiting gap H1 is located at a position where the inner wall of the inner tube 120 is spaced apart from the outer wall of the energy transmission element 110 by a smallest distance in the initial state. The energy transmission element 110 is offset relative to the inner tube 120 to some extent under the effect of an external force, but an offset value is relatively small due to the first limiting gap H1. Through the above structure, it is able to ensure that the energy transmission element 110 is coaxial with the inner tube 120 as possible even in the case of the external force, thereby to ensure the straightness of a part of the energy transmission element 110 extending into a to-be-ablated tissue 900 to a greatest extent. Even if the inner wall of the inner tube 120 is in contact with the outer wall of the energy transmission element 110 under the effect of the external force, it is also able to ensure that the cooling medium flows stably between the inner wall of the inner tube 120 and the outer wall of the energy transmission element 110.

In some embodiments of the present disclosure, in the initial state, the outer tube 130 is arranged coaxially with the inner tube 120, no contact point is formed between the inner wall of the outer tube 130 and the outer wall of the inner tube 120, a cross section of the outer wall of the inner tube 120 has a shape different from a cross section of the inner wall of the outer tube 130, a plurality of second limiting gaps H2 is arranged circumferentially between the inner wall of the outer tube 130 and the outer wall of the inner tube 120, and the second limiting gap H2 is located at a position where the inner wall of the outer tube 130 is spaced apart from the outer wall of the inner tube 120 by a smallest distance in the initial state. The inner tube 120 is offset relative to the outer tube 130 to some extent under the effect of an external force, but an offset value is relatively small due to the second limiting gap H2. Through the above structure, it is able to ensure that the inner tube 120 is coaxial with the outer tube 130 as possible even in the case of the external force, thereby to ensure the uniformity of the second channel T2 between the inner tube 120 and the outer tube 130 to a greatest extent. Even if the inner wall of the outer tube 130 is in contact with the outer wall of the outer tube 120 under the effect of the external force, it is also able to ensure that the cooling medium flows stably between the inner wall of the outer tube 130 and the outer wall of the inner tube 120.

In some illustrative embodiments of the present disclosure, the plurality of first contact portions is arranged circumferentially between the inner wall of the inner tube 120 and the outer wall of the energy transmission element 110, and the plurality of second contact portions M2 is arranged circumferentially between the inner wall of the outer tube 130 and the outer wall of the inner tube 120. In some embodiments of the present disclosure, in the initial state, the energy transmission element 110 is arranged coaxially with the inner tube 120, there is no contact point between the inner wall of the inner tube 120 and the outer wall of the energy transmission element 110, the outer tube 130 is arranged coaxially with the inner tube 120, and there is no contact point between the inner wall of the outer tube 130 and the outer wall of the inner tube 120. In some other embodiments of the present disclosure, the plurality of first contact portions M1 is arranged circumferentially between the inner wall of the inner tube 120 and the outer wall of the energy transmission element 110, and in the initial state, the outer tube 130 is arranged coaxially with the inner tube 120 and there is no contact point between the inner wall of the outer tube 130 and the outer wall of the inner tube 120. Alternatively, in some embodiments of the present disclosure, in the initial state, the energy transmission element 110 is arranged coaxially with the inner tube 120, there is no contact point between the inner wall of the inner tube 120 and the outer wall of the energy transmission element 110, and the plurality of second contact portions M2 is arranged circumferentially between the inner wall of the outer tube 130 and the outer wall of the inner tube 120.

As shown in FIGs. 1 to 3, in some embodiments of the present disclosure, the ablation catheter 100 further includes a first adapter assembly 140 and a second adapter assembly 150. The energy transmission element 110 passes through the first adapter assembly 140, the inner tube 120 passes through the second adapter assembly 150, a portion of the outer tube 130 extends into the second adapter assembly 150, a proximal end of the outer tube 130 extends beyond a proximal end of the second adapter assembly 150, and a distal end of the inner tube 120 extends to a distal end of the second adapter assembly 150.

The distal end of the second adapter assembly 150 is detachably coupled to a proximal end of the first adapter assembly 140. One of the first adapter assembly 140 and the second adapter assembly 150 is in communication with the cooling source 200, and the cooling medium from the cooling source 200 passes through the first channel T1 and the second channel T2 and is then discharged from the ablation catheter via the other of the first adapter assembly 140 and the second adapter assembly 150. In a possible embodiment of the present disclosure, the first channel T1 is in communication with the cooling source 200 via the first adapter assembly 140, and the cooling medium in the second channel T2 is discharged via the second adapter assembly 150. In FIG. 3, a flow direction of the cooling medium is indicated by arrows.

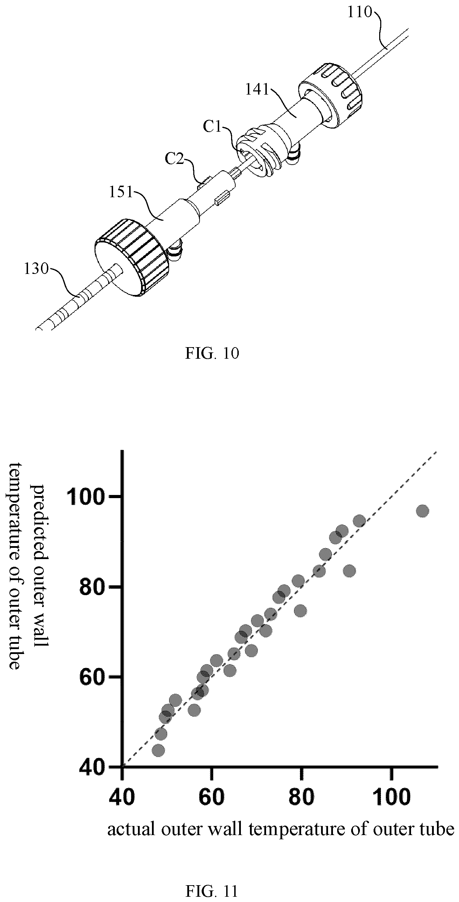

FIG. 10 is an exploded view of the second adapter assembly and the first adapter assembly according to one embodiment of the present disclosure. In the embodiment of the present disclosure, a rotation limiting structure is provided between the second adapter assembly 150 and the first adapter assembly 140, and configured to limit rotation of the second adapter assembly 150 relative to the first adapter assembly 140.

In the context, the term "proximal end" refers to an end of a component arranged close to or facing the to-be-ablated tissue 900 in a case that the ablation catheter 100 is in an operating state, and the term "distal end" refers to an end of a component arranged away from or facing away from the to-be-ablated tissue 900 in a case that the ablation catheter 100 is in the operating state.

A proximal end of the energy transmission element 110 and a proximal end of the inner tube 120 both extend into the hermetically sealed member 131, and there are a gap between the proximal end of the energy transmission element 110 and an inner wall of the hermetically sealed member 131 and a gap between the proximal end of the inner tube 120 and the inner wall of the hermetically sealed member 131. The cooling medium from the cooling source 200 passes through the first channel T1 to the proximal end of the energy transmission element 110, so as to cool the proximal end of the energy transmission element 110. After the heat exchange at the proximal end of the energy transmission element 110, the cooling medium passes through the second channel T2 to a medium recycling pool 300 coupled to the second adapter assembly 150.

According to the ablation catheter 100 in the embodiments of the present disclosure, the energy transmission element 110 passes through the first adapter assembly 140, the inner tube 120 passes through the second adapter assembly 150, a part of the outer tube 130 extends into the second adapter assembly 150, and the proximal end of the outer tube 130 extends beyond the proximal end of the second adapter assembly 150. The distal end of the second adapter assembly 150 is detachably coupled to the proximal end of the first adapter assembly 140, so as to facilitate the assembling of the ablation catheter 100. The rotation limiting structure is arranged between the second adapter assembly 150 and the first adapter assembly 140, and configured to limit the rotation of the second adapter assembly 150 relative to the first adapter assembly 140, so as to prevent the second adapter assembly 150 from rotating relative to the first adapter assembly 140 after the assembling, thereby to ensure the accurate position relationship between the second adapter assembly 150 and the first adapter assembly 140 the accurate position relationship among the energy transmission element 110, the inner tube 120 and the outer tube 130.

In some embodiments of the present disclosure, the hermetically sealed member 131 of the outer tube 130 is of a semi-spherical structure to facilitate the flow of the cooling medium and prevent the occurrence of turbulent flow. In some embodiments of the present disclosure, through simulating a flow field in the ablation catheter 100, sizes and relative positions of the members of the ablation catheter 100 may be optimized, so as to remarkably prevent the heat accumulation at the proximal end of the ablation catheter 100 due to the turbulent flow, thereby to protect the ablation catheter 100 in a more stable manner in the case of thermocoagulation.

Each of the inner tube 120 and the outer tube 130 may be made of glass having high light transmittance, so as to improve the ablation efficiency. In some other embodiments of the present disclosure, each of the inner tube 120 and the outer tube 130 may also be made of resin.

In some embodiments of the present disclosure, the distal end of the second adapter assembly 150 is inserted into the proximal end of the first adapter assembly 140. The proximal end of the first adapter assembly 140 is provided with a guiding groove C1 extending in a direction parallel to an axial direction of the first adapter assembly 140. An outer peripheral surface of the second adapter assembly 150 is provided with a guiding protrusion C2 adapted to the guiding groove C1, and the guiding protrusion C2 is inserted into the guiding groove C1 to limit the rotation of the second adapter assembly 150 relative to the first adapter assembly 140.

In the embodiments of the present disclosure, the rotation limiting structure includes the guiding groove C1 and the guiding protrusion C2 matching each other. In a case that the second adapter assembly 150 is assembled with the first adapter assembly 140, the guiding protrusion C2 is guided and limited by the guiding groove C1, so as to prevent the rotation of the second adapter assembly 150 relative to the first adapter assembly 140, thereby to prevent the second adapter assembly 150 from being offset relative to the first adapter assembly 140.

In some embodiments of the present disclosure, there are at least two guiding grooves C1 arranged around a central axis of the first adapter assembly 140, and there are at least two guiding protrusions C2 corresponding to the guiding grooves C1 respectively.

In the embodiment of the present disclosure, there are two guiding grooves C1 arranged symmetrically, and there are two guiding protrusions C2 arranged symmetrically on the outer peripheral surface of the second adapter assembly 150. In some other embodiments of the present disclosure, one or more than two guiding grooves C1 and one or more than two guiding protrusions C2 may be provided.

In some embodiments of the present disclosure, the ablation catheter 100 further includes a connection member 160 sleeved onto the distal end of the second adapter assembly 150 and detachably coupled to the proximal end of the first adapter assembly 140. The connection member 160 is configured to detachably couple the distal end of the second adapter assembly 150 to the proximal end of the first adapter assembly 140.

In the embodiment of the present disclosure, the connection member 160 is a threaded connection member, which is in threaded connection with the proximal end of the first adapter assembly 140. In a case that the proximal end of the first adapter assembly 140 is gradually screwed into the connection member 160, the second adapter assembly 150 and the first adapter assembly 140 are locked together. In the embodiment of the present disclosure, through the connection member 160, the distal end of the second adapter assembly 150 is detachably coupled to the proximal end of the first adapter assembly 140, so as to facilitate the assembling of the ablation catheter 100, and prevent the energy transmission element 110 from being damaged due to complex assembling. In addition, in a case that the connection member 160 is a threaded connection member, it is able to control the connection tightness between the second adapter assembly 150 and the first adapter assembly 140 more easily, and facilitate the disassembling and adjustment.

In some embodiments of the present disclosure, the distal end of the second adapter assembly 150 is inserted into the proximal end of the first adapter assembly 140. A first central guiding structure D1 is arranged in a cavity of the first adapter assembly 140, and a second central guiding structure D2 is arranged at the distal end of the second adapter assembly 150. In a state where the second adapter assembly 150 is coupled to the first adapter assembly 140 through the connection member 160, the second central guiding structure D2 cooperates with the first central guiding structure D1 in such a manner that a central axis of the second adapter assembly 150 coincides with a central axis of the first adapter assembly 140.

In the above embodiment of the present disclosure, through the cooperation of the second central guiding structure D2 with the first central guiding structure D1, it is able to further ensure that the second adapter assembly 150 is arranged coaxially with the first adapter assembly 140 after the second adapter assembly 150 is coupled to the first adapter assembly 140 through the connection member 160, thereby to further ensure that the energy transmission element 110, the inner tube 120 and the outer tube 130 are arranged coaxially.

In some embodiments of the present disclosure, the first central guiding structure D1 and the second central guiding structure D2 have conical surfaces matching each other.

In some other embodiments of the present disclosure, the first central guiding structure D1 and the second central guiding structure D2 may be any other structures cooperating with each other for guiding. For example, the first central guiding structure D1 and the second central guiding structure D2 have step-like surfaces matching each other.

In some embodiments of the present disclosure, the first adapter assembly 140 includes a first cylinder 141, the second adapter assembly 150 includes a second cylinder 151 and an inner tube sealing member 152 located at a distal end of the second cylinder 151, the first central guiding structure D1 is arranged on an inner peripheral surface of the first cylinder 141, and the second central guiding structure D2 is arranged on an outer peripheral surface of the inner tube sealing member 152.

In some embodiments of the present disclosure, the inner tube sealing member 152 is an elastic member. In the state where the second adapter assembly 150 is coupled to the first adapter assembly 140 through the connection member 160, the second central guiding structure D2 on the outer peripheral surface of the inner tube sealing member 152 is pressed against the first central guiding structure D1 on the inner peripheral surface of the first cylinder 141.

In some embodiments of the present disclosure, the inner tube sealing member 152 is a soft member made of silica gel.

In the above embodiment of the present disclosure, in a case that the inner tube sealing member 152 is an elastic member and the second central guiding structure D2 on the outer peripheral surface of the inner tube sealing member 152 is pressed against the first central guiding structure D1 on the inner peripheral surface of the first cylinder 141, it is able to improve a center-alignment effect of the second adapter assembly 150 and the first adapter assembly 140 while ensuring a sealing effect of the inner tube sealing member 152.

As shown in FIGs. 1 and 2, in some embodiments of the present disclosure, the first adapter assembly 140 includes a first cylinder 141, a sealing plug 142 and a sealing cover 143. The energy transmission element 110 passes through the first cylinder 141, and a first interface member 1411 extends from the first cylinder 141. In the embodiment of the present disclosure, the first interface 1411 is in communication with the cooling source 200. The sealing cover 143 is configured to press the sealing plug 142 against a distal end of the first cylinder 141. The energy transmission element 110 passes through the sealing cover 143 and the sealing plug 142, and is in hermetical engagement with the sealing plug 142. In some embodiments of the present disclosure, the sealing cover 143 is in threaded connection with the distal end of the first cylinder 141. In some other embodiments of the present disclosure, the sealing cover 143 is detachably coupled to the distal end of the first cylinder 141 in any other ways.

In some embodiments of the present disclosure, the sealing plug 142 is a soft member made of silica gel.

As shown in FIGs. 1 and 2, in some embodiments of the present disclosure, the second adapter assembly 150 includes a second cylinder 151, an inner tube sealing member 152, an outer tube sealing member 153, and an outer tube fixation member 154. The inner tube 120 passes through the second cylinder 151, a part of the outer tube 130 extends into the second cylinder 151, and a second interface member 1511 extends from the second cylinder 151 and is in communication with the second channel T2. The inner tube sealing member 152 is arranged at a distal end of the second cylinder 151, and the outer tube sealing member 153 is arranged at a proximal end of the second cylinder 151. The outer tube fixation member 154 is configured to press the outer tube sealing member 153 against the proximal end of the second cylinder 151, and couple the outer tube 130 to the second cylinder 151 coaxially. In some embodiments of the present disclosure, the outer tube fixation member 154 is detachably coupled to the proximal end of the second cylinder 151 in any other ways.

In some embodiments of the present disclosure, the outer tube sealing member 153 is a soft member made of silica gel.

For example, the cooling medium from the cooling source 200 sequentially passes through the first channel T1 and the second channel T2 and is then discharged from the ablation catheter. In the design of the ablation catheter 100, usually an effective flow area of the first channel T1 needs to be the same as an effective flow area of the second channel T2, so as to provide a smaller flow resistance. In the embodiments of the present disclosure, the ablation catheter 100 further includes the first adapter assembly 140, the second adapter assembly 150 and the connection member 160. In a case that the first adapter assembly 140, the second adapter assembly 150 and the connection member 160 are coupled to such components as the inner tube 120 and the outer tube 130 and in a case that the ablation catheter 100 is coupled to the other surgical tools (e.g., a guiding screw), the outer tube 130 may be subjected to a radial pressure, so the effective flow area of the second channel T2 may be reduced. In some embodiments of the present disclosure, the effective flow area of the second channel T2 is 1.1 to 1.2 times of the effective flow area of the first channel T1. In this way, it is able to compensate for the reduced effective flow area of the outer tube due to the radial pressure, and maintain the flow resistance in the ablation catheter at a low level, thereby to improve the flow efficiency of the cooling medium and the operating performance of the ablation catheter.

In the embodiment of the present disclosure, the energy transmission element 110 is coupled to a laser source, and has a light-exiting member facing the hermetically sealed member 131.

In the embodiments of the present disclosure, the cooling medium passes through the first channel T1 and the second channel T2 and is then discharged from the ablation catheter, so as to form a closed-loop system for the cooling medium. In the closed-loop system, flow distribution of the cooling medium is critical to an operating temperature of the proximal end of the ablation catheter 100 (i.e., an end where the hermetically sealed member 131 is located), and the treatment security and effectiveness may be influenced thereby.

There exists the following relationship between the cooling medium and heat: the flow of the cooling medium should be large enough to prevent a temperature of the proximal end of the ablation catheter 100 from being too high under the effect of the heat generated by the laser beam. In a cooling process of the energy transmission element 110, actually the cooling medium may form a laminar flow at a surface of the energy transmission element 110. At this time, the heat transfer efficiency may be influenced by a temperature gradient.

The temperature gradient refers to a temperature change in a unit length. In the cooling system, in a case that the temperature gradient is larger, it means that the heat may be transferred from a heated object to the cooling medium more rapidly.

The heat transfer efficiency may be improved through increasing the temperature gradient, a heat transfer area or a heat transfer coefficient. In the ablation catheter 100, the cooling medium may be normal saline (or carbon dioxide in some other embodiments of the present disclosure). The temperature gradient in the ablation catheter 100 mainly depends on laser power as well as a thermal capacity and a flow rate of the cooling medium.

At the proximal end of the ablation catheter 100, convective heat transfer is mainly used due to a narrow space. In an actual cooling process, a large temperature gradient needs to be formed between the cooling medium and a surface of the heated object as possible. Hence, the flow rate and the flow quantity of the cooling medium in the ablation catheter 100 need to be set in such a manner as to cool the ablation catheter 100 and maintain a constant temperature difference.

In the ablation catheter 100, the effective flow area of the first channel T1 is influenced by a diameter of the energy transmission element 110, a minimum diameter of each of the inner tube 120 and the outer tube 130 is limited by the diameter of the energy transmission element 110, and a flow path and flow efficiency of the cooling medium depend on sizes of the inner tube 120 and the outer tube 130. Generally, in a case that each of the inner tube 120 and the outer tube 130 is provided with a large diameter, it is able to improve a cooling effect in a better manner. Hence, the diameters of the inner tube 120 and the outer tube 130 need to be set in such a manner as to provide a sufficient cooling capacity and prevent the occurrence of a large puncture injury due to the oversized ablation catheter 100.

To be specific, a minimum sectional area of the inner tube 120 is directly influenced by the diameter of the energy transmission element 110. The larger the diameter of the energy transmission element 110, the larger the diameter of the inner tube 120, so as to facilitate the insertion and fixation of the energy transmission element 110. The flow rate and the flow quantity of the cooling medium depend on the effective flow area of the first channel T1 and the effective flow area of the second channel T2. In a case of a large effective flow area, the flow quantity may increase, and thereby the cooling efficiency may be improved. In a case of a small effective flow area, the flow resistance and an intensity of pressure may increase, and thereby a large pumping pressure needs to be provided. In a case that the diameter of the outer tube 130 is too large, a risk of injury to a puncture path may increase, and the surgical safety and a patient's comfort level may be adversely affected. Hence, the size of the ablation catheter 100 needs to be designed in such a manner as to make a compromise between the cooling effect and the operation convenience.

In some embodiments of the present disclosure, the energy transmission element 110 is coupled to the laser source. The size of the ablation catheter is determined through obtaining diameter information about the outer tube 130 and the inner tube 120 based on laser energy of the laser source and target temperature information about the ablation catheter 100 after determining the diameter of the energy transmission element 110. In some embodiments of the present disclosure, the laser energy of the laser source may be represented by laser power.

Further, in some embodiments of the present disclosure, the obtaining the diameter information about the outer tube 130 and the inner tube 120 based on the laser energy of the laser source and the target temperature information about the ablation catheter 100 includes Step S110 to Step S130.

In Step S110, a flow quantity corresponding to the target temperature information is obtained based on the laser power of the laser source, the target temperature information about the ablation catheter 100 and a first fitting model. The first fitting model includes a mapping relation between temperature information about the ablation catheter 100 and the laser power as well as the flow quantity.

In Step S120, the diameter information about the outer tube 130 is obtained based on the flow quantity and a second fitting model. The second fitting model includes a mapping relation among the flow quantity, an intensity of pressure and the diameter information about the outer tube 130, and the diameter information about the outer tube 130 includes an outer diameter and an inner diameter of the outer tube 130. In the embodiment of the present disclosure, the intensity of pressure is an intensity of pressure of the cooling medium at an inlet.

In Step S130, the dimeter information about the inner tube 120 is based on the outer diameter of the outer tube 130.

Based on the above, in some embodiments of the present disclosure, the energy transmission element 110 is provided with a light-exiting member facing the hermetically sealed member 131. The temperature information about the ablation catheter 100 includes an outer wall temperature of the outer tube 130 and a core temperature of the light-exiting member. In some embodiments of the present disclosure, the target temperature information includes a target temperature of the outer wall of the outer tube which is smaller than or equal to 90°C. In some other embodiments of the present disclosure, the target temperature of the outer wall of the outer tube is within a range of 80°C to 95°C.

In some embodiments of the present disclosure, Computational Fluid Dynamics (CFD) software is used to simulate the flow of the cooling medium in a case of different diameters of the outer tube 130 and the inner tube 120, and analyze a temperature field distribution, so as to obtain a first fitting model.

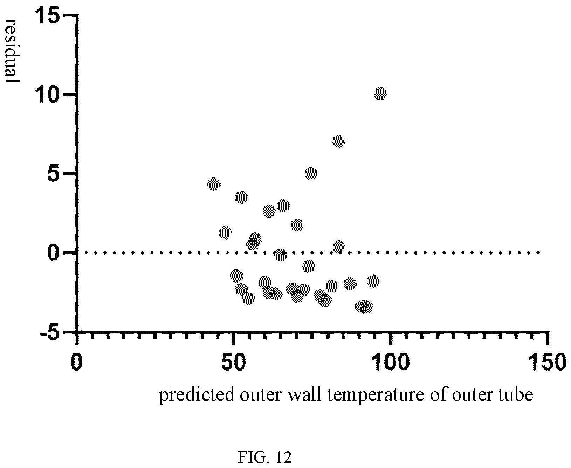

The first fitting model includes a first sub-model and a second sub-model. The first sub-model is used to represent a mapping relation between the outer wall temperature of the outer tube and the laser power as well as the flow quantity, and the second sub-model is used to represent a mapping relation between the core temperature of the light-exiting member and the laser power as well as the flow quantity. In the embodiment of the present disclosure, the first sub-model and the second sub-model are obtained through multiple linear regression fitting. FIG. 11 is a diagram of predicted outer wall temperatures of the outer tube in the first sub-model obtained through multiple linear regression fitting using simulation software according to one embodiment of the present disclosure, and FIG. 12 is a diagram of residuals of outer wall temperatures of the outer tube in the first sub-model obtained through multiple linear regression fitting using simulation software according to one embodiment of the present disclosure. In some embodiments of the present disclosure, the first sub-model obtained through fitting is Tx1=31.92+4.424*Px-0.7383*Qx, where Tx1 represents the outer wall temperature of the outer tube in unit of °C, Px represents the laser power in unit of watt (W), and Qx represents the flow quantity in unit of mL/min.

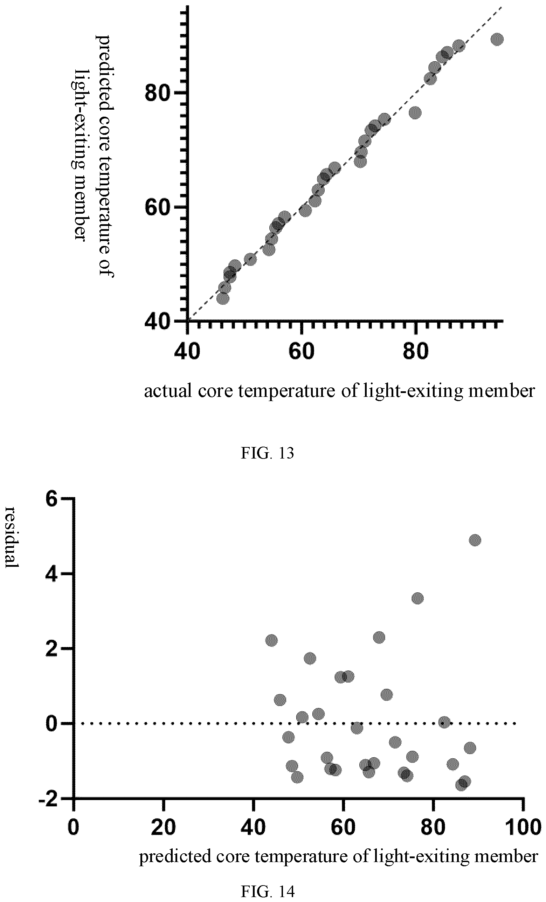

FIG. 13 is a diagram of predicted core temperatures of the light-exiting member in the second sub-model obtained through multiple linear regression fitting using simulation software according to one embodiment of the present disclosure, and FIG. 14 is a diagram of residuals of the core temperatures of the light-exiting member in the second sub-model obtained through multiple linear regression fitting using simulation software according to one embodiment of the present disclosure. In some embodiments of the present disclosure, the second sub-model obtained through fitting is Tx2=26.00+4.272*Px-0.3809*Qx, where Tx2 represents the core temperature of the light-exiting member in unit of °C, Px represents the laser power in unit of W, and Qx represents the flow quantity in unit of mL/min.

In the first sub-model and the second sub-model obtained through fitting, considering a laser transmission loss, the fitting is performed in a case that the laser transmission efficiency is 90%. Of course, in some other embodiments of the present disclosure, the fitting may also be performed in a case that the laser transmission efficiency has the other values.

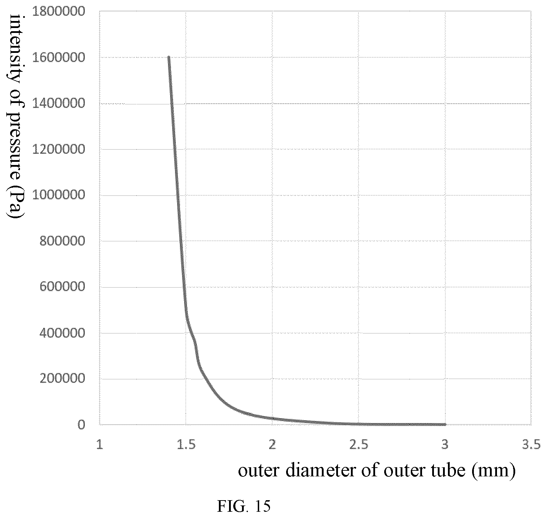

The second fitting model may be obtained through fitting after multiple data measurements. FIG. 15 is a fitted curve diagram of a second fitting model according to one embodiment of the present disclosure. For example, in a case that the flow quantity is 20mL/min, a correspondence between the intensity of pressure and the diameter information about the outer tube 130 is shown in Table 1.

**Table 1**

| Outer diameter of outer tube (mm) | Inner diameter of outer tube (mm) | Intensity of pressure (Pa) |
|---|---|---|
| 1.4 | 1.1 | 1600000 |
| 1.5 | 1.2 | 500000 |
| 1.55 | 1.25 | 364000 |
| 1.6 | 1.3 | 220000 |
| 1.8 | 1.5 | 62000 |
| 2.3 | 2 | 8920 |
| 3 | 2.7 | 1747 |

Based on Table 1, a mapping relation between the intensity of pressure and the diameter information about the outer tube 130 in a case that the flow quantity is 20mL/min is shown in FIG. 15. In Step S 110, after obtaining the flow quantity corresponding to the target temperature information, the diameter information about the outer tube 130 is obtained based on the flow quantity in combination with the second fitting model and the required intensity of pressure. The diameter information about the outer tube 130 matches the diameter information about the inner tube 120, so the diameter information about the inner tube 120 may be obtained based on the diameter information about the outer tube 130. In this way, it is able to accurately determine the diameter information about the outer tube 130 and the inner tube 120 according to the practical need.

In some embodiments of the present disclosure, in order to meet the outer wall temperature of the outer tube and the core temperature of the light-exiting member, in a case that two different flow quantity estimates are calculated through the first sub-model and the second sub-model, a relationship between the two flow quantity estimates needs to be taken into consideration, so as to determine the flow quantity. To be specific, the flow quantity may be determined using a mean value method, a weighting method, a mathematical optimization algorithm (e.g., linear regression, nonlinear regression or genetic algorithm), or iterative optimization.

The present disclosure further provides in some embodiments an ablation system, which includes the above-mentioned ablation catheter 100, a cooling source 200 and a medium recycling pool 300.

The ablation catheter 100 includes an energy transmission element 110, an inner tube 120 and an outer tube 130. The energy transmission element 110 is configured to transmit a laser signal. The inner tube 120 is sleeved onto the energy transmission element 110, and a first channel T1 is formed between an inner wall of the inner tube 120 and an outer wall of the energy transmission element 110. The outer tube 130 is sleeved onto the inner tube 120, a second channel T2 is formed between an inner wall of the outer tube 130 and an outer wall of the inner tube 120, the outer tube 130 is provided with a hermetically sealed member 131 at a proximal end, and the second channel T2 is in communication with the first channel T1 in the hermetically sealed member 131. The first channel T1 or the second channel T2 is in communication with the cooling source 200 for providing a cooling medium, e.g., a cooling liquid, and the cooling medium from the cooling source 200 passes through the first channel T1 and the second channel T2 and is then discharged from the ablation catheter. In any cross section including the energy transmission element 110, the inner tube 120 and the outer tube 130, any virtual line extending from a center of the energy transmission element 110 to the outer wall of the outer tube 130 passes through at least one of the first channel T1 or the second channel T2.

The cooling source 200 is in communication with the first channel T1 or the second channel T2 of the ablation catheter 100, and the medium recycling pool 300 is in communication with the second channel T2 or the first channel T1 of the ablation catheter 100. In a possible embodiment of the present disclosure, the cooling source 200 is in communication with the first channel T1 of the ablation catheter 100, and the medium recycling pool 300 is in communication with the second channel T2 of the ablation catheter 100.

According to the ablation system in the embodiments of the present disclosure, the ablation catheter 100 includes the energy transmission element 110, the inner tube 120 and the outer tube 130, the first channel T1 is formed between the inner wall of the inner tube 120 and the outer wall of the energy transmission element 110, and the second channel T2 is formed between the inner wall of the outer tube 130 and the outer wall of the inner tube 120. In any cross section including the energy transmission element 110, the inner tube 120 and the outer tube 130, any virtual line extending from the center of the energy transmission element 110 to the outer wall of the outer tube 130 passes through at least one of the first channel T1 or the second channel T2. In a case that the ablation catheter 100 is in an operating state, the cooling medium from the cooling source 200 passes through the first channel T1 and the second channel T2, and there is the cooling medium on any virtual line extending from the center of the energy transmission element 110 to the outer wall of the outer tube 130, i.e., the cooling medium exists in any radial direction of the ablation catheter 100. In this way, it is able to reduce the temperature, and prevent the ablation catheter 100 from being overheated in a case that the energy transmission element 110, the outer tube 130 and the inner tube 120 are in contact with each other at a same position, thereby to prevent the ablation catheter 100 from being damaged, and improve a service life and reliability of the ablation catheter 100.

In some embodiments of the present disclosure, the ablation catheter 100 further includes a first adapter assembly 140 and a second adapter assembly 150. The energy transmission element 110 passes through the first adapter assembly 140, the inner tube 120 passes through the second adapter assembly 150, a portion of the outer tube 130 extends into the second adapter assembly 150, a proximal end of the outer tube 130 extends beyond a proximal end of the second adapter assembly 150, and a distal end of the inner tube 120 extends to a distal end of the second adapter assembly 150. The distal end of the second adapter assembly 150 is detachable coupled to a proximal end of the first adapter assembly 140. One of the first adapter assembly 140 and the second adapter assembly 150 is in communication with the cooling source 200, and the cooling medium from the cooling source 200 passes through the first channel T1 and the second channel T2 and is then discharged from the ablation catheter via the other of the first adapter assembly 140 and the second adapter assembly 150. In the embodiment of the present disclosure, the first channel T1 is in communication with the cooling source 200 via the first adapter assembly 140, and the cooling medium in the second channel T2 is discharged via the second adapter assembly 150.

In a possible embodiment of the present disclosure, the cooling source 200 is coupled to the first adapter assembly 140 of the ablation catheter 100, and the medium recycling pool 300 is coupled to the second adapter assembly 150 of the ablation catheter 100.

According to the ablation system in the embodiments of the present disclosure, the inner tube 120 passes through the second adapter assembly 150, a part of the outer tube 130 extends into the second adapter assembly 150, and the proximal end of the outer tube 130 extends beyond the proximal end of the second adapter assembly 150. The distal end of the second adapter assembly 150 is detachably coupled to the proximal end of the first adapter assembly 140, so as to facilitate the assembling of the ablation catheter 100. A rotation limiting structure is arranged between the second adapter assembly 150 and the first adapter assembly 140, and configured to limit the rotation of the second adapter assembly 150 relative to the first adapter assembly 140, so as to prevent the second adapter assembly 150 from rotating relative to the first adapter assembly 140 after the assembling, thereby to ensure the accurate position relationship between the second adapter assembly 150 and the first adapter assembly 140 the accurate position relationship among the energy transmission element 110, the inner tube 120 and the outer tube 130.

In some embodiments of the present disclosure, the cooling source 200 includes a cooling source tank 210 and a pump 220. The cooling source tank 210 is in communication with a first interface member 1411 of the first adapter assembly 140 via an inlet pipeline 400, and the pump 220 is located on the inlet pipeline 400. For example, the pump 220 is a peristaltic pump.

In some embodiments of the present disclosure, the medium recycling pool 300 is in communication with a second interface member 1511 of the second adapter assembly 150 via an outlet pipeline 500.

In some embodiments of the present disclosure, the cooling source 200 further includes a heater 230 arranged at the cooling source tank 210. In a possible embodiment of the present disclosure, the heater 230 is arranged outside the cooling source tank 210. The heater 230 is configured to heat the cooling medium to an appropriate temperature, so as to prevent a tissue from being irritated in a case that the cooling medium flows through the tissue.

In some embodiments of the present disclosure, the ablation system further includes a laser source, a temperature sensor, a control unit and an adjustment unit. The laser source is configured to output a laser beam to the laser transmission element 100, so as to perform laser ablation on a to-be-ablated tissue. The temperature sensor is configured to monitor in real time a temperature of the ablation catheter, so as to obtain target temperature information. The control unit is configured to control the output of the laser beam and the supply of the cooling medium. The adjustment unit is configured to dynamically adjust in real time the supply of the cooling medium using a first fitting model in response to the target temperature information about the ablation catheter and the laser beam from the laser source, so as to control the temperature of the ablation catheter. The first fitting model includes a mapping relation between temperature information about the ablation catheter and laser power of the laser source as well as a flow quantity.

In some embodiments of the present disclosure, the energy transmission element 110 is provided with a light-exiting member facing the hermetically sealed member 131. The temperature information about the ablation catheter 100 includes an outer wall temperature of the outer tube 130 and a core temperature of the light-exiting member.

In some embodiments of the present disclosure, CFD software is used to simulate the flow of the cooling medium in a case of different diameters of the outer tube 130 and the inner tube 120, and analyze a temperature field distribution, so as to obtain a first fitting model. In some embodiments of the present disclosure, the first fitting model includes a first sub-model and a second sub-model. The first sub-model is used to represent a mapping relation between the outer wall temperature of the outer tube and the laser power as well as the flow quantity, and the second sub-model is used to represent a mapping relation between the core temperature of the light-exiting member and the laser power as well as the flow quantity. In a possible embodiment of the present disclosure, the first sub-model and the second sub-model are obtained through multiple linear regression fitting.

In some embodiments of the present disclosure, the first sub-model is Tx1=31.92+4.424*Px-0.7383*Qx, where Tx1 represents the outer wall temperature of the outer tube in unit of °C, Px represents the laser power in unit of W, and Qx represents the flow quantity in unit of mL/min.

In some embodiments of the present disclosure, the second sub-model obtained through fitting is Tx2=26.00+4.272*Px-0.3809*Qx, where Tx2 represents the core temperature of the light-exiting member in unit of °C, Px represents the laser power in unit of W, and Qx represents the flow quantity in unit of mL/min.

In the first sub-model and the second sub-model obtained through fitting, considering a laser transmission loss, the fitting is performed in a case that the laser transmission efficiency is 90%. Of course, in some other embodiments of the present disclosure, the fitting may also be performed in a case that the laser transmission efficiency has the other values.

The above embodiments are for illustrative purposes only, but the present disclosure is not limited thereto. Obviously, a person skilled in the art may make further modifications and improvements without departing from the spirit of the present disclosure, and these modifications and improvements shall also fall within the scope of the present disclosure.

## Claims

1. An ablation catheter, comprising:
an energy transmission element;
an inner tube sleeved onto the energy transmission element, a first channel being formed between an inner wall of the inner tube and an outer wall of the energy transmission element; and
an outer tube sleeved onto the inner tube, a second channel being formed between an inner wall of the outer tube and an outer wall of the inner tube,
wherein the outer tube is provided with a hermetically sealed member at a proximal end, the second channel is in communication with the first channel in the hermetically sealed member, the first channel or the second channel is in communication with a cooling source, and a cooling medium from the cooling source passes through the first channel and the second channel and is discharged from the ablation catheter,
wherein in any cross section comprising the energy transmission element, the inner tube and the outer tube, any virtual line extending from a center of the energy transmission element to the outer wall of the outer tube passes through at least one of the first channel or the second channel.

2. The ablation catheter according to claim 1, wherein in an initial state, the energy transmission element is arranged coaxially with the inner tube, no contact point is formed between the inner wall of the inner tube and the outer wall of the energy transmission element, a cross section of the energy transmission element has a shape different from a cross section of the inner wall of the inner tube, a plurality of first limiting gaps is arranged circumferentially between the inner wall of the inner tube and the outer wall of the energy transmission element, and the first limiting gap is located at a position where the inner wall of the inner tube is spaced apart from the outer wall of the energy transmission element by a smallest distance in the initial state.

3. The ablation catheter according to claim 1, wherein in an initial state, the outer tube is arranged coaxially with the inner tube, no contact point is formed between the inner wall of the outer tube and the outer wall of the inner tube, a cross section of the outer wall of the inner tube has a shape different from a cross section of the inner wall of the outer tube, a plurality of second limiting gaps is arranged circumferentially between the inner wall of the outer tube and the outer wall of the inner tube, and the second limiting gap is located at a position where the inner wall of the outer tube is spaced apart from the outer wall of the inner tube by a smallest distance in the initial state.

4. The ablation catheter according to claim 1, further comprising:
a first adapter assembly, the energy transmission element passing through the first adapter assembly; and
a second adapter assembly, the inner tube passing through the second adapter assembly, a part of the outer tube extending into the second adapter assembly, the proximal end of the outer tube extending beyond a proximal end of the second adapter assembly, a distal end of the inner tube extending to a distal end of the second adapter assembly, and the distal end of the second adapter assembly being detachably coupled to a proximal end of the first adapter assembly,
wherein one of the first adapter assembly and the second adapter assembly is in communication with the cooling source, the cooling medium from the cooling source passes through the first channel and the second channel and is discharged from the ablation catheter via the other of the first adapter assembly and the second adapter assembly;
wherein a rotation limiting structure is provided between the second adapter assembly and the first adapter assembly, and configured to limit rotation of the second adapter assembly relative to the first adapter assembly.

5. The ablation catheter according to claim 4, wherein the distal end of the second adapter assembly is inserted into the proximal end of the first adapter assembly, the proximal end of the first adapter assembly is provided with a guiding groove extending in a direction parallel to an axial direction of the first adapter assembly, an outer peripheral surface of the second adapter assembly is provided with a guiding protrusion adapted to the guiding groove, and the guiding protrusion is inserted into the guiding groove to limit the rotation of the second adapter assembly relative to the first adapter assembly.

6. The ablation catheter according to claim 4, wherein the distal end of the second adapter assembly is inserted into the proximal end of the first adapter assembly, a first central guiding structure is arranged in a cavity of the first adapter assembly, a second central guiding structure is arranged at the distal end of the second adapter assembly, and in a state where the second adapter assembly is coupled to the first adapter assembly through a connection member, the second central guiding structure cooperates with the first central guiding structure in such a manner that a central axis of the second adapter assembly coincides with a central axis of the first adapter assembly.

7. The ablation catheter according to claim 4, wherein the first adapter assembly comprises:
a first cylinder, the energy transmission element passing through the first cylinder, a first interface member extending from the first cylinder;
a sealing plug; and
a sealing cover configured to press the sealing plug against a distal end of the first cylinder,
wherein the energy transmission element passes through the sealing cover and the sealing plug, and is in hermetical engagement with the sealing plug.

8. The ablation catheter according to claim 4, wherein the second adapter assembly comprises:
a second cylinder, the inner tube passing through the second cylinder, a part of the outer cylinder extending into the second cylinder, a second interface member extending from the second cylinder and in communication with the second channel;
an inner tube sealing member arranged at a distal end of the second cylinder;
an outer tube sealing member arranged at a proximal end of the second cylinder; and
an outer tube fixation member configured to press the outer tube sealing member against the proximal end of the second cylinder, and couple the outer tube to the second cylinder coaxially.

9. The ablation catheter according to claim 1, wherein the energy transmission element is coupled to a laser source, and a size of the ablation catheter is determined through obtaining diameter information about the outer tube and the inner tube based on laser energy of the laser source and target temperature information about the ablation catheter after determining a diameter of the energy transmission element.

10. The ablation catheter according to claim 9, wherein the obtaining the diameter information about the outer tube and the inner tube based on the laser energy of the laser source and the target temperature information about the ablation catheter comprises:
obtaining a flow quantity corresponding to the target temperature information about the ablation catheter based on laser power of the laser source, the target temperature information and a first fitting model, the first fitting model comprising a mapping relation between temperature information about the ablation catheter and the laser power as well as the flow quantity;
obtaining the diameter information about the outer tube based on the flow quantity and a second fitting model, the second fitting model comprising a mapping relation among the flow quantity, an intensity of pressure and the diameter information about the outer tube, the diameter information about the outer tube comprising an outer diameter and an inner diameter of the outer tube; and
obtaining the dimeter information about the inner tube based on the outer diameter of the outer tube.

11. The ablation catheter according to claim 10, wherein the energy transmission element comprises a light-exiting member facing the hermetically sealed member, the temperature information about the ablation catheter comprises an outer wall temperature of the outer tube and a core temperature of the light-exiting member, and the first fitting model comprises a first sub-model and a second sub-model,
wherein the first sub-model is Tx1=31.92+4.424*Px-0.7383*Qx, where Tx1 represents the outer wall temperature of the outer tube, Px represents the laser power and Qx represents the flow quantity; and
the second sub-model is Tx2=26.00+4.272*Px-0.3809*Qx, where Tx2 represents the core temperature of the light-exiting member, Px represents the laser power, and Qx represents the flow quantity.

12. The ablation catheter according to claim 11, wherein the target temperature information comprises a target temperature of the outer wall of the outer tube which is smaller than or equal to 90°C.

13. The ablation catheter according to claim 1, wherein an effective flow area of the second channel is 1.1 to 1.2 times of an effective flow area of the first channel.

14. An ablation system, comprising:
the ablation catheter according to any one of claims 1 to 13;
a cooling resource in communication with the first channel or the second channel of the ablation catheter; and
a medium recycling pool in communication with the second channel or the first channel of the ablation catheter.

15. The ablation system according to claim 14, further comprising:
a laser source configured to output a laser beam to the laser transmission element, so as to perform laser ablation on a to-be-ablated tissue;
a temperature sensor configured to monitor in real time a temperature of the ablation catheter, so as to obtain target temperature information;
a control unit configured to control the output of the laser beam and the supply of a cooling medium; and
an adjustment unit configured to dynamically adjust in real time the supply of the cooling medium using a first fitting model in response to the target temperature information about the ablation catheter and the laser beam from the laser source, so as to control the temperature of the ablation catheter,
wherein the first fitting model comprises a mapping relation between temperature information about the ablation catheter and laser power of the laser source as well as a flow quantity.

16. The ablation system according to claim 15, wherein the energy transmission element comprises a light-exiting member, the temperature information about the ablation catheter comprises an outer wall temperature of the outer tube and a core temperature of the light-exiting member, and the first fitting model comprises a first sub-model and a second sub-model,
wherein the first sub-model is Tx1=31.92+4.424*Px-0.7383*Qx, where Tx1 represents the outer wall temperature of the outer tube, Px represents the laser power and Qx represents the flow quantity; and
the second sub-model is Tx2=26.00+4.272*Px-0.3809*Qx, where Tx2 represents the core temperature of the light-exiting member, Px represents the laser power, and Qx represents the flow quantity.
